# EUROPEAN PATENT SPECIFICATION

(11) **EP 3 951 341 B1**
(45) Date of publication and mention of the grant of the patent: **29.03.2023**
(21) Application number: 20189497.9
(22) Date of filing: 05.08.2020
(51) Int. Cl.: G01K 11/24, G01N 29/024, G01N 29/14

(54) **TEMPERATURE MEASUREMENT**
TEMPERATURMESSUNG
MESURE DE TEMPÉRATURE

(43) Date of publication of application: 09.02.2022
(73) Proprietor: Nokia Technologies Oy, 02610 Espoo (FI)
(72) Inventor: CRONIN, Harry, Cambridge, CB1 3DE (GB)
(74) Representative: Swindell & Pearson Limited

(56) References cited:
- DE-A1-102011 056 533
- JP-A- 2017 003 318

## Description

### TECHNOLOGICAL FIELD

Embodiments of the present disclosure relate to temperature measurement. Some relate to exhaled breath temperature measurement.

### BACKGROUND

The speed of sound can be estimated using a difference in the time of arrival of sound at two microphones located at different distances from the sound source. This can be used to determine the temperature of a medium through which the sound passes since speed of sound has a known relationship with temperature. As a matter of example, reference is made to JP2017003318 A and DE102011056533 A1 which both disclose temperature determination based on speed of sound.

### BRIEF SUMMARY

According to the claimed invention there is provided an apparatus comprising means for: determining a time difference between arrival at a device of a sound produced by a sound source and an electromagnetic signal reflected from a surface, of a body comprising the sound source, which vibrates when the sound is produced; and providing the time difference to enable determination of a fluid temperature based on the speed of sound through the fluid between the body and the device.

According to the claimed invention there is provided a method comprising: determining a time difference between arrival at a device of a sound produced by a sound source and an electromagnetic signal reflected from a surface, of a body comprising the sound source, which vibrates when the sound is produced; and providing the time difference to enable determination of a fluid temperature based on the speed of sound through the fluid between the body and the device.

According to the claimed invention there is provided a computer program comprising instructions that, when the program is run on a computer, cause the computer to perform: determining a time difference between arrival at a device of a sound produced by a sound source and an electromagnetic signal reflected from a surface, of a body comprising the sound source, which vibrates when the sound is produced; and providing the time difference to enable determination of a fluid temperature based on the speed of sound through the fluid between the body and the device.

Advantageous features are claimed in the appended claims.

### BRIEF DESCRIPTION

Some examples will now be described with reference to the accompanying drawings in which:
FIG 1 shows an example of the disclosure;
FIG 2 shows an example apparatus;
FIG 3 shows an example method; and
FIGS 4A and 4B show an example application.

### DETAILED DESCRIPTION

Sound is a vibration of any frequency, not just those which are within audible limits of human hearing, that propagates through a medium at the speed of sound in that medium.

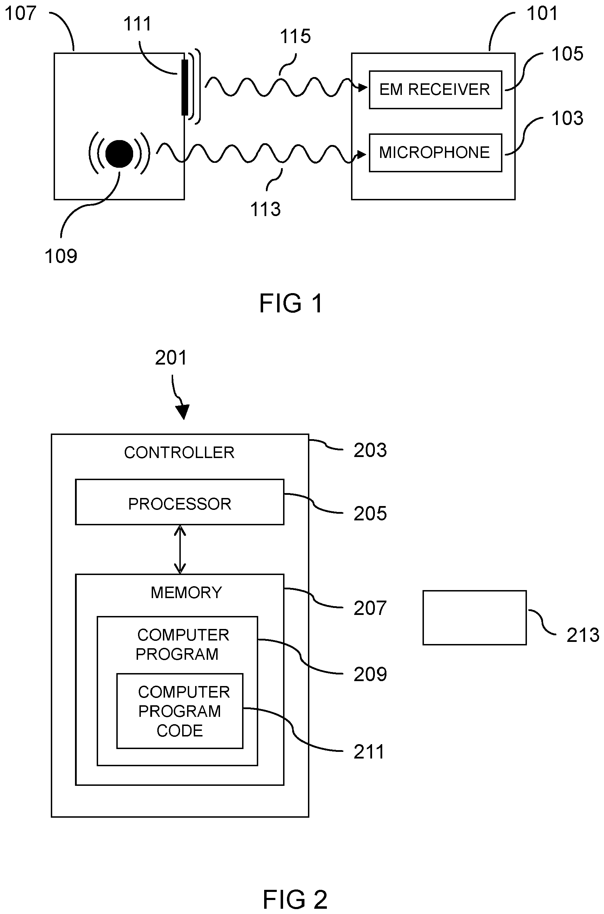

Sound 113 produced at a sound source 109, as shown in FIG 1, can be detected via vibration induced at a device 101 which is remote from a body 107 comprising the sound source 109. The sound 113 can be detected using, for example, one or more microphones 103 of the device 101.

Where a sound 113 is produced by a sound source 109 comprised in a body 107, a surface 111 of that body 107 will vibrate with at least similar frequency characteristics as vibrations at the device 101. In some examples, the vibrating surface 111 can be the sound source 109 or part of the sound source 109. In other examples, the surface 111 can be distinct from the sound source 109.

The vibration of the surface 111 of the body 107 can be measured using an electromagnetic signal 115 reflected from that surface 111 which propagates through the medium separating the body 107 and the device 101 at the speed of light in that medium. The electromagnetic signal 115 can be detected using, for example, an electromagnetic receiver (or transceiver) 105 such as one or more antennas (or antenna array) or one or more image sensors.

Thus, a signal comprising information, obtained via vibration of the surface 111, regarding the sound 113 produced by the sound source 109 will arrive at the device 101 before the sound 113 itself arrives at the device 101. The electromagnetic signal 115 reflected from the surface 111 enables a lookahead measurement of the sound 113, though it is to be appreciated that the information about the sound 113 may not be extracted until after the sound 113 itself has arrived at the device 101 whether because signal processing is deliberately delayed, because of limitations in the processing speed, or otherwise.

This difference in the time of arrival at the device 101 can be used to determine the speed of sound in the medium. The speed of sound in a medium has a known relationship with temperature, though the exact relationship differs according to multiple factors including the medium - for example, in relatively slow-moving air, the relationship can be approximated as *c*² = 403*T*, where c is the speed of sound in m/s and T is the temperature in K. Consequently, this difference in the time of arrival at the device 101 can be used to determine the temperature of the medium through which the sound 113 travels from the body 107 to the device 101.

Examples of the present disclosure relate to an apparatus 201 for enabling a measurement of this temperature by determining this difference in the time of arrival at the device 101.

FIG 2 schematically illustrates an apparatus 201 according to examples of the disclosure. The apparatus 201 illustrated in FIG 2 may be a chip or a chip-set. In some examples the apparatus 201 may be or be comprised within the device 101. The device 101 may be a user device such as a mobile phone or another portable computing device. In some examples the apparatus 201 could be provided in another device such as a server or processing device that is separate to the device 101.

In the example of FIG 2 the apparatus 201 comprises a controller 203. Implementation of the controller 203 may be as controller circuitry. The controller 203 may be implemented in hardware alone, have certain aspects in software including firmware alone or can be a combination of hardware and software (including firmware).

As illustrated in FIG 2 the controller 203 may be implemented using instructions that enable hardware functionality, for example, by using executable instructions of a computer program 209 in a general-purpose or special-purpose processor 205 that may be stored on a computer readable storage medium (disk, memory, etc.) to be executed by such a processor 205.

The processor 205 is configured to read from and write to the memory 207. The processor 205 may also comprise an output interface via which data and/or commands are output by the processor 205 and an input interface via which data and/or commands are input to the processor 205.

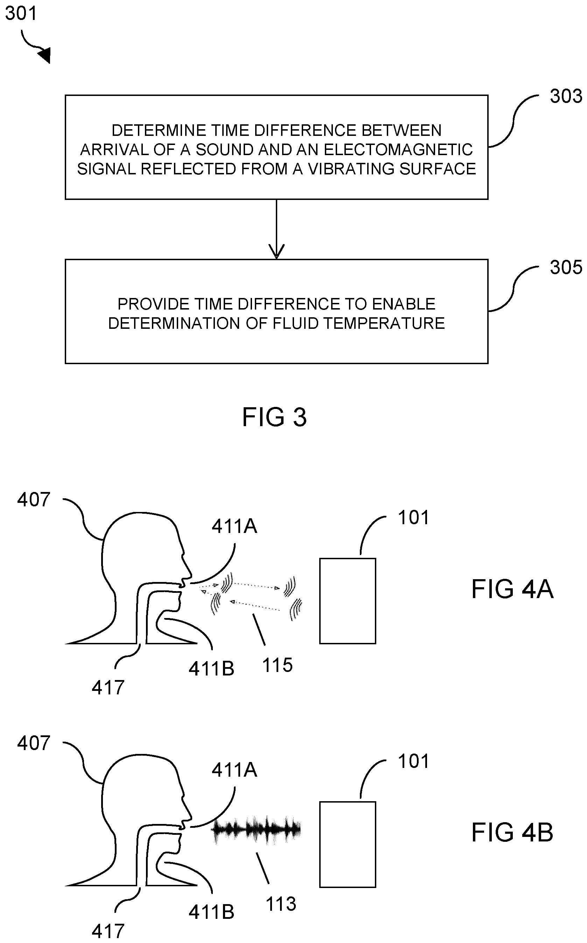

The memory 207 stores a computer program 209 comprising computer program instructions (computer program code 211) that controls the operation of the apparatus 201 when loaded into the processor 205. The computer program instructions, of the computer program 209, provide the logic and routines that enables the apparatus 201 to perform the method 301 illustrated in FIG 3. The processor 205 by reading the memory 207 is able to load and execute the computer program 209.

The apparatus 201 therefore comprises: at least one processor 205; and at least one memory 207 including computer program code 211, the at least one memory 207 and the computer program code configured to, with the at least one processor 205, cause the apparatus 201 at least to perform:
determining 303 a time difference between arrival at a device 101 of a sound 113 produced by a sound source 109 and an electromagnetic signal 115 reflected from a surface 111, of a body 107 comprising the sound source 109, which vibrates when the sound 113 is produced; and
providing 305 the time difference to enable determination of a fluid temperature based on the speed of sound through the fluid between the body 107 and the device 101.

As illustrated in FIG 2, the computer program 209 may arrive at the apparatus 201 via any suitable delivery mechanism 213. The delivery mechanism 213 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 209. The delivery mechanism may be a signal configured to reliably transfer the computer program 209. The apparatus 201 may propagate or transmit the computer program 209 as a computer data signal. In some examples the computer program 209 may be transmitted to the apparatus 201 using a wireless protocol such as Bluetooth, Bluetooth Low Energy, Bluetooth Smart, 6LoWPan (IPv6 over low power personal area networks) ZigBee, ANT+, near field communication (NFC), Radio frequency identification, wireless local area network (wireless LAN) or any other suitable protocol.

In some examples there is provided computer program instructions for causing an apparatus 201 to perform at least the following:
determining 303 a time difference between arrival at a device 101 of a sound 113 produced by a sound source 109 and an electromagnetic signal 115 reflected from a surface 111, of a body 107 comprising the sound source 109, which vibrates when the sound 113 is produced; and
providing 305 the time difference to enable determination of a fluid temperature based on the speed of sound through the fluid between the body 107 and the device 101.

The computer program instructions may be comprised in a computer program 209, a non-transitory computer readable medium, a computer program product, a machine readable medium. In some but not necessarily all examples, the computer program instructions may be distributed over more than one computer program 209.

Although the memory 207 is illustrated as a single component/circuitry it may be implemented as one or more separate components/circuitry some or all of which may be integrated/removable and/or may provide permanent/semi-permanent/ dynamic/cached storage.

Although the processor 205 is illustrated as a single component/circuitry it may be implemented as one or more separate components/circuitry some or all of which may be integrated/removable. The processor 205 may be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in FIG 3 may represent steps in a method and/or sections of code in the computer program 209. The illustration of a particular order to the blocks does not necessarily imply that there is a required or preferred order for the blocks and the order and arrangement of the block may be varied. Furthermore, it may be possible for some blocks to be omitted.

FIG 3 shows an example method 301 that can be implemented by the apparatus 201 as shown in FIG 2.

The method 301 comprises, at block 303, determining a time difference between arrival at the device 101 of the sound 113 produced by the sound source 109 and the electromagnetic signal 115 reflected from the surface 111, of the body 107 comprising the sound source 109, which vibrates when the sound 113 is produced. It is to be appreciated that the time difference may be expressed as a quantity of time or as a quantity having a known relationship with time such as a frequency.

In some examples, the method 301 comprises causing guidance to be provided to a user of the device 101. The guidance may indicate a target relative position between the body 107 and the device 101. The target relative position may comprise a suitable angle and distance from the body 107 for the purpose of detecting the sound 113 and the electromagnetic signal 115. The target relative position can ensure that there is a direct line-of-sight between the device 101 and the surface 111 which vibrates when the sound 113 is produced.

In examples where the temperature to be determined is that of a fluid expelled from the body 107, the user of the device 101 can be guided to position the device 101 such that the sound 113 arriving at the device 101 will propagate through the expelled fluid. The user of the device 101 can be guided to position the device 101 such that the sound 113 arriving at the device 101 has travelled along the substantially the same path as travelled by the fluid after expulsion from the body 107.

The target relative position may therefore be determined based on a location of the opening in the body 107 from which said fluid is expelled. The location of this opening may be detected using for example an infrared sensor, computer vision, or any other suitable means.

In comparison to a direct temperature measurement of said fluid as it may arrive at the device 101, a determination of temperature by the present method 301 can be less affected by cooling of the fluid outside of the body 107. For example, a gas expelled from the body 107 at relatively low velocity may not arrive at the device 101 at a temperature which significantly differs from the ambient temperature.

In some examples, the user of the device 101 can be guided to position the device 101 such that it has a direct line-of-sight to a surface 111 which is proximate the sound source 109 and which vibrates when the sound 113 is produced. The electromagnetic signal 115 can be reflected from this proximate surface 111. The surface 111 may be sufficiently proximate the sound source 109 that the distance travelled by the electromagnetic signal 115 reflected from the surface 111 to the device 101 and the distance travelled by the sound 113 from the sound source 109 to the device 101 are the same or substantially the same, to the extent that the difference that any discrepancy in distance makes to the determination of temperature is less than the target sensitivity of the temperature determination.

In some examples the device 101 both emits an electromagnetic signal 115 which is reflected from the surface 111 and detects this electromagnetic signal 115 as reflected from the surface 111. In other examples the electromagnetic signal 115 which is reflected from the surface 111 may be emitted by another device and the device 101 may detect the electromagnetic signal 115 reflected from the surface 111. Thus, the method 301 can comprise causing the device 101 to detect the electromagnetic signal 115 reflected from the surface 111 and can comprise causing the device 101 to emit the electromagnetic signal 115 which is subsequently reflected from the surface 111.

In some examples the electromagnetic signal 115 is a short wavelength radio wave including radiation in the Extremely high frequency (EHF) band such as a millimetre wave (mmWave), having wavelengths from approximately ten to one millimetre, or radiation in the Super high frequency (SHF) band such as a centimetre wave, having wavelengths from approximately ten to one centimetre. Short wavelength radio waves have strong reflectance properties and are thus effective at inferring small amplitude vibrations such as those likely to be induced at the surface 111 of the body 107 when the sound 113 is produced by the sound source 109. Use of higher frequency electromagnetic radiation, including light perceivable by a human eye, is also possible. The radiation used for the electromagnetic signal 115 may have high temporal coherence.

In some examples emitting the electromagnetic signal 115 may be conditional on a relative position between the body and the device corresponding with the aforementioned target relative position.

An electrical signal representing the vibration of the surface 111 can be obtained by transducing the electromagnetic signal 115 reflected from the surface into an electrical signal and processing this electrical signal to filter out frequency components of the electromagnetic signal 115 as they were prior to reflection from the surface 111, for example as originally transmitted. This electrical signal can be processed to determine one or more features of the vibration of the surface 111 such as frequency components or time-domain waveforms.

An electrical signal representing the sound 113 can be obtained by transducing the sound 113 arriving at the device 101. This electrical signal can be processed to determine one or more features of the sound 113 such as frequency components or time-domain waveforms.

The time difference can be determined using one or more features of the sound 113 and one or more features of the vibration of the surface 111 which are identified to correspond to each other. These one or more features may be identified by comparing an electrical signal representing the sound 113 and an electrical signal representing the vibration of the surface 111 using correlations, pattern recognition or any other suitable process. This can be performed in, for example, the time domain, frequency domain, or in a joint time-frequency domain.

In some examples the one or more features comprise one or more chirps. A chirp is a signal in which the frequency either increases over time or in which the frequency decreases over time. Suitable chirps can be linear or non-linear chirps. For example, quadratic chirps or exponential chirps may also be suitable.

Many sounds can be represented as superpositions of chirps. The method 301 can comprise analysis of the electrical signals representing the sound 113 and the vibration of the surface 111 to identify and extract one or more chirps. Extracting these chirps enables the use of chirp mixing. Chirp mixing is a signal processing technique by which the resolution of the time difference (or temporal resolution more generally) can be improved beyond the limitations imposed by the sampling rate of the microphone 103 of the device 101. Improving the resolution of the time difference improves the resolution of the fluid temperature determination.

Chirp mixing can exceed the sampling rate limit of the microphone 103 because it changes the problem of directly measuring a time difference into a problem of comparing frequency components. Frequency components of a suitable chirp will fall within the measurable range of the microphone 103. Chirp mixing compares these frequency components of the electrical signal representing the vibration of the surface 111 with those of the electrical signal representing the sound 113 in such a way that the time difference information is turned into a frequency somewhere within this range after suitable processing.

Both the sound 113 and the vibration of the surface 111 can be measured over the same time window, starting when a suitable chirp is detected in electrical signals representing the vibration of the surface 111 and stopping at the end of that chirp. Compared with the vibration of the surface 111, a certain range of the time-base will be clipped from the sound 113, leading to a certain range of frequencies being "missing". The missing frequency range will depend on the time difference. When the two signals are mixed, low-pass filtered and the time-derivative of the mixed signal is taken, the resulting signal will show a frequency peak corresponding to the range of frequencies which were present in both the sound 113 and the vibration of the surface 111 during the time window. This remaining frequency therefore gives a sensitive measure of the time difference between arrival at the device 101 of the sound 113 and the electromagnetic signal 115 reflected from the vibrating surface 111.

Accordingly, in some examples the time difference is determined from the frequency of a signal resulting from chirp mixing using a chirp extracted from the signal representing vibration of the surface 111 and a corresponding chirp extracted from the signal representing the sound 113.

Where multiple chirps can be identified in the signals, the chirp used in the chirp mixing may be that which is determined to have the largest bandwidth or largest bandwidth in which a suitable mathematical frequency-time relation is maintained.

The resolution of the time difference can be also improved beyond the limitations imposed by the sampling rate of the microphone of the device 101 by means other than chirp mixing such as phase-based sampling methods. The time it takes for the sound 113 to propagate to the device 101 introduces a fixed delay to all frequency components, as compared to the corresponding frequency components of the electromagnetic signal 115. This fixed delay causes a frequency-dependent phase shift in the electrical signal representing the sound 113 as compared to the electrical signal representing the vibration of the surface 111. By measuring the phase angle between the two electrical signals as a function of frequency, while the distance between the device 101 and the body 107 remains constant, the time difference between arrival at the device 101 of the sound 113 and the electromagnetic signal 115 reflected from the vibrating surface 111 can be calculated.

At block 305 of the method 301, the time difference is provided to enable determination of a fluid temperature based on the speed of sound through the fluid between the body 107 and the device 101.

The time difference is, in some examples, provided to means for determining the fluid temperature. Said means can comprise a calculation module of the apparatus 201 or device 101 which is configured to calculate the fluid temperature based on the provided time difference. The calculation module can for example be implemented as code in the computer program 109. In other examples, the time difference is provided to another device, separate to the apparatus 201 or the device 101, such as a server or remote processing device, at which the calculation of the fluid temperature may be performed.

Accordingly, the method 301 may comprise determining the temperature of the fluid between the body 107 and the device 101. Alternatively, the method 301 may end at the point of providing the time difference to the means for determining the fluid temperature or subsequently proceed from this point with receipt of data indicating the fluid temperature which has been determined elsewhere.

Determining the fluid temperature further comprises obtaining a distance between the body 107 and the device 101. In conjunction with the time difference, the distance enables the speed of sound through the fluid between the body 107 and the device 101 to be determined. For greater accuracy in the determination of the fluid temperature, the distance between the surface 111 and the device 101 can be obtained.

The distance can be obtained by: receiving data indicating the distance from another device capable of measuring the distance between the body 107 or surface 111 and the device 101; processing a signal, such as the electromagnetic signal 115 reflected from the surface 111, to determine the distance; or by otherwise measuring or causing the device 101 to measure the distance using, for example, other sensors such as Time-of-flight (ToF) sensors, laser diodes for range finding, or a camera and suitable image processing to infer distance based on depth of field from the camera.

To determine the distance between the surface 111 and the device 101 using the electromagnetic signal 115 reflected from the surface 111, the electromagnetic signal 115 may be transmitted from the device 101 in the form of a pulse. In another example, the electromagnetic signal 115 can be a frequency-modulated continuous-wave.

By using the electromagnetic signal 115 reflected from the surface 111 to determine the distance between the surface 111 and the device 101, the distance at the time the sound 113 is produced can be obtained, providing good accuracy in the determination of the speed of sound. As an alternative, however, determination of the distance from the electromagnetic signal 115 can be made using the part of the signal corresponding to reflection from the surface 111 just before the vibrations induced by the sound 113 begin or just after they cease, which will also give good accuracy on the presumption that the relative position of the body 107 and device 101 does not greatly vary during the production of the sound 113.

The method 301 can comprise causing an indication of the determined fluid temperature to be provided to a user of the device 101.

Blocks 303 and 305 of the method 301 may not be separate or discrete steps in the method 301 or separate or discrete sections of code in the computer program 209. In some examples, signals representing the sound 113 and the vibrations of the surface 111 may be provided as inputs and the fluid temperature may be output. Thus, the method 301 can be considered, in some examples, as determining a temperature of a fluid, between a device 101 and a body 107 comprising a source 109 of a sound 113, based on: a distance between the device 101 and the body 107; and a time difference between arrival at the device 101 of the sound 113 and an electromagnetic signal 115 reflected from a surface 111, of the body 107, which vibrates when the sound 113 is produced.

The hereinbefore described apparatus 201 and method 301 find application in for example air temperature measurements. In some examples, the electromagnetic signal 115 can be reflected from a loudspeaker, for example from the diaphragm of the loudspeaker, in order to obtain a lookahead measurement of the sound produced by the loudspeaker and accordingly to determine a temperature of the air between the loudspeaker and the device 101 as described in the foregoing disclosure.

Other applications can relate to measurement of industrial equipment or machinery which produce both heated air and sound. For example, the body 107 may be an oven and the foregoing disclosure may be applied to determine the temperature of air within the oven. In another example, the body 107 may be an exhaust and the foregoing disclosure may be applied to determine the temperature of exhaust gases.

In some examples the hereinbefore described apparatus 201 and method 301 find application in monitoring exhaled breath temperature of a subject 407, as shown in the example of FIGS 4A and 4B.

Exhaled breath temperature monitoring is an established medical intervention for a range of conditions, especially asthma, and initial results have indicated it may also serve as an early marker of lung cancer. Early-stage medical research has indicated exhaled breath temperature monitoring could aid detection of febrile illness, with exhaled breath temperature rises of 1-2 °C measured in rhinovirus and influenza patients being detected anywhere up to 24 hours before detectable body temperature rises.

In the example application of FIGS 4A and 4B, the sound 113 is a sound produced using a subject's vocal tract 409 and the surface 111 is a body part 411A,B of the subject 407 which vibrates when the sound is produced using the subject's vocal tract 409.

Accordingly, in the example application of FIGS 4A and 4B, block 303 of the method 301 comprises determining a time difference between arrival at the device 101 of a sound 113 produced using a subject's vocal tract 409 (as shown in FIG 4B) and an electromagnetic signal 115 reflected from a body part 411A,B of the subject 407 which vibrates when the sound 113 is produced using the subject's vocal tract 409 (as shown in FIG 4A). Block 305 correspondingly comprises providing the time difference to enable determination of the subject's exhaled breath temperature based on the speed of sound through the air between the subject 407 and the device 101.

Sounds which can be produced using a subject's vocal tract 409, where the subject is a human, include, for example, voiced and unvoiced speech, singing, laughing, crying, screaming, clicks, whistling, and whispering. In the production of such sounds, the vocal tract 409 may not be the primary sound source 109 but may nevertheless be used in filtering the sound 113.

The sound 113 can be a sound produced by an egressive airstream, however it is not limited in this manner. Since the sound 113 can travel faster than exhaled breath, even sounds that are produced by an ingressive airstream will pass through a subject's previously exhaled breath column to arrive at the device 101. Accordingly, the exhaled breath temperature can be determined using both egressive and ingressive sounds.

The sound 113 produced using the subject's vocal tract 409 propagates as vibrations through the subject's body and can be measured as vibrations of a surface, for example in areas proximate the vocal tract 409 such as the throat 411B or mouth 411A.

If the subject's exhaled breath temperature is outside a threshold, then an alert can be caused to be provided to the subject. The threshold can be determined from a record of one or more previously determined exhaled breath temperatures. It is to be appreciated, however, that the threshold may not be determined from a record of previously determined exhaled breath temperatures as abnormal breath temperature is a stable threshold factor across human populations. Accordingly, ad-hoc determination of exhaled breath temperature can be performed without need for regular monitoring.

In some examples guidance which indicates a target sound to be produced can be caused to be provided to the subject 407. The target sound can be one capable of being detected by the microphone 103 of the device 101 and capable of inducing vibration of the body part 411A,B that can be detected using the electromagnetic signal 115.

The target sound may be a sound which can be decomposed into a summation of chirps, whether linear or non-linear or both, to enable chirp mixing as described in the foregoing.

It is to be appreciated that this is inessential. In some examples the sampling rate of the microphone 103 of the device 101 is sufficiently high that chirp mixing is not required. In some examples, other means for improving the resolution of the time difference beyond the limitations imposed by the sampling rate of the microphone of the device 101 can be used.

The target sound may also specify a volume or range of volumes. Since air speed, particularly high air speed, can be an influence on the temperature measurement, it is disadvantageous if the subject 407 produces the sound 113 with too forceful exhalation. Thus, to achieve slower moving air, the target sound may guide the subject 407 to produce the sound 113 at a lower volume.

The mouth's soft palate provides a relatively wide range of line-of-sight viewing angles when the mouth 411A is open and is thus an appropriate surface 111 at which to measure the vibration induced by production of the sound 113. Thus, the subject 407 may be guided to produce sounds which can be made with an open mouth, or to avoid sounds which cannot be made with an open mouth.

In some examples, the subject 407 may be provided with no initial guidance in regards to what sound 113 to produce. Where chirp mixing may be used, the method 301 can make use of opportune chirps in the subject's natural speech or other sound production using their vocal tract 409. If there is no suitable chirp in the sound 113 made by subject 407, the subject 407 may be instructed to produce another sound 113 or may be guided to produce a target sound as described in the foregoing.

Ambient air temperature can affect the temperature of exhaled breath, though within the variation of indoor room temperature, the effect is minimal. Thus, the subject 407 may be guided to use the device 101 indoors. In other examples, the ambient air temperature can be controlled for by performing the method 301 in respect of sounds which arrive at the device 101 without passing through the subject's exhaled breath column. For example, when sound 113 is produced using the subject's vocal tract 409, the subject's chest wall (not shown) may also vibrate, causing corresponding vibration at the skin of the chest. The chest vibrations are not affected by vocal tract-resonances and, thus, can be decomposed from the sound 113. The chest sound component arrives at the device 101 without having passed through the subject's exhaled breath column. A temperature of the ambient air is therefore derivable from measuring the time difference between arrival at the device 101 of this chest sound component and an electromagnetic signal reflected from the vibrating skin of the chest. Where the electromagnetic receiver (or transceiver) 105 can resolve incoming signals into their directions-of-arrival, these measurements for determining ambient air temperature can be performed contemporaneously with those measurements for determining exhaled breath temperature. An alternative approach to measuring sounds which arrive at the device 101 without passing through the subject's exhaled breath column is to change the position of the device 101 so that it is no longer aligned with the subject's exhaled breath column. In this new position, the same vibrating surface 111 may still be measured, or another vibrating surface, such as the skin of the chest, may be measured. In yet other examples, ambient air temperature can be obtained from other sensors capable of measuring air temperature.

A number of other physiological processes or behaviours can also affect exhaled breath temperature. For example, recent exercise, particularly strenuous exercise, can cause a rise in exhaled breath temperature of around 1 °C which persists for about an hour. Food or alcohol consumption, hay fever, and several others factors can also cause a rise in exhaled breath temperature, though these are usually responsible for a rise in exhaled breath temperature of 0.5 °C or less. To mitigate these confounding factors, implementation of the method 301 can be avoided during or during a period after exercise or food and drink consumption, for example.

By way of an illustrative example, for a subject 407 having a healthy exhaled breath temperature of 307 K (about 34 °C), the speed of sound through their breath column will be around 352 m/s in accordance with the aforementioned relationship *c*² = 403T. If the device 101 is held 0.5 m from the subject 407, the time difference between arrival at the device 101 of the sound 113 produced using the subject's vocal tract 409 and the electromagnetic signal 115 reflected from the vibrating body part 411A,B of the subject 407 will be around 1.422 ms. A rise in the exhaled breath temperature to 309 K (about 36 °C; a rise of 2 °C), which may trigger an alert to the subject 407, causes the sound 113 to travel faster towards the device 101 at a speed of around 353 m/s. In this instance, the device 101 still being held 0.5 m from the subject 407, the time difference will decrease by around 5 µs to 1.417 ms. If the sampling interval of the microphone 103 is greater than 5 µs (a sampling rate of around 217 kHz), then signal processing techniques such as chirp mixing can be used to provide the resolution required to measure such rises in exhaled breath temperature.

Where a structural feature has been described, it may be replaced by means for performing one or more of the functions of the structural feature whether that function or those functions are explicitly or implicitly described.

Consequently, in some examples, the apparatus 201 comprises means for: determining 303 a time difference between arrival at a device 101 of a sound 113 produced by a sound source 109 and an electromagnetic signal 115 reflected from a surface 111, of a body 107 comprising the sound source 109, which vibrates when the sound 113 is produced; and providing 305 the time difference to enable determination of a fluid temperature based on the speed of sound through the fluid between the body 107 and the device 101.

In some but not necessarily all examples, the apparatus 201 is configured to communicate data from the apparatus 201 with or without local storage of the data in a memory 207 at the apparatus 201 and with or without local processing of the data by circuitry or processors at the apparatus 201.

The data may, for example, be the determined time difference.

The data may be stored in processed or unprocessed format remotely at one or more devices. The data may be stored in the Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus 201 may comprise a communications interface such as, for example, a radio transceiver for communication of data.

The apparatus 201 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The processing of the data, whether local or remote, may produce an output - for example, the fluid temperature or exhaled breath temperature. The output may be communicated to the apparatus 201 where it may produce an output sensible to the subject such as an audio output, visual output or haptic output or cause the device 101 to produce said output.

The recording of data may comprise only temporary recording, or it may comprise permanent recording or it may comprise both temporary recording and permanent recording, Temporary recording implies the recording of data temporarily. This may, for example, occur during sensing or image capture, occur at a dynamic memory, occur at a buffer such as a circular buffer, a register, a cache or similar. Permanent recording implies that the data is in the form of an addressable data structure that is retrievable from an addressable memory space and can therefore be stored and retrieved until deleted or over-written, although long-term storage may or may not occur.

As used here "module" refers to a unit or apparatus that excludes certain parts or components that would be added by an end manufacturer or a user.

## Claims

1. An apparatus **characterized by** means for:
determining a time difference between arrival at a device (101) of a sound (113) produced by a sound source (109) and an electromagnetic signal (115) reflected from a surface (111), of a body (107) comprising the sound source (109), which vibrates when the sound (113) is produced; and
providing the time difference to enable determination of a fluid temperature based on the speed of sound through the fluid between the body (107) and the device (101).

2. The apparatus of claim 1 wherein the time difference is determined using one or more features of the sound (113) and one or more features of a signal representing the vibration of the surface (111) which are identified to correspond to each other.

3. The apparatus of claim 2 wherein the signal representing the vibration of the surface (111) is obtained by transducing the electromagnetic signal (115) reflected from the surface (111) into an electrical signal and processing this electrical signal to filter out frequency components of the electromagnetic signal (115) prior to reflection from the surface (111).

4. The apparatus of any preceding claim wherein the time difference is determined from a frequency of a signal resulting from chirp mixing using a chirp extracted from the signal representing vibration of the surface (111) and a corresponding chirp extracted from the sound (113).

5. The apparatus of any preceding claim wherein the means are configured to obtain a distance between the body (107) and the device (101).

6. The apparatus of claim 5 wherein the distance is obtained by processing of the electromagnetic signal (115) reflected by the surface (111).

7. The apparatus of any preceding claim wherein the means are configured to cause guidance to be provided to a user of the device (101) which indicates a target relative position between the body (107) and the device (101).

8. The apparatus of any preceding claim wherein the means are configured to cause the device (101) to emit an electromagnetic signal (115) which is reflected from the surface (111) and to detect the electromagnetic signal (115) reflected from the surface (111).

9. The apparatus of any preceding claim wherein the means are configured to transduce the sound (113) into an electrical signal representing the sound (113).

10. The apparatus of any preceding claim wherein the sound (113) is a sound produced using a subject's vocal tract (409) and the surface is a body part (411A, B) of the subject (407) which vibrates when the sound (113) is produced using the subject's vocal tract (409).

11. The apparatus of claim 10 wherein the means are configured to provide the time difference to enable determination of the subject's exhaled breath temperature based on the speed of sound through the air between the subject (407) and the device (101).

12. The apparatus of claim 11 wherein the means are configured to cause an alert to be provided to the subject (407) if the subject's exhaled breath temperature is outside a threshold determined from a record of one or more previously determined exhaled breath temperatures.

13. The apparatus of any of claims 10 to 12 wherein the means are configured to cause guidance to be provided to the subject (407) which indicates a target sound to be produced.

14. A method **characterized by** the following steps:
determining (303) a time difference between arrival at a device (101) of a sound (113) produced by a sound source (109) and an electromagnetic signal (115) reflected from a surface (111), of a body (107) comprising the sound source (109), which vibrates when the sound (113) is produced; and
providing (305) the time difference to enable determination of a fluid temperature based on the speed of sound through the fluid between the body (107) and the device (101).

15. A computer program comprising instructions that, when the program is run on a computer, cause the computer to perform the following steps:
determining a time difference between arrival at a device (101) of a sound (113) produced by a sound source (109) and an electromagnetic signal (115) reflected from a surface (111), of a body (107) comprising the sound source (109), which vibrates when the sound (113) is produced; and
providing the time difference to enable determination of a fluid temperature based on the speed of sound through the fluid between the body (107) and the device (101).

## Patentansprüche

1. Einrichtung, die durch Mittel gekennzeichnet ist zum:
Bestimmen einer Zeitdifferenz zwischen einer Ankunft eines Tons (113), der von einer Tonquelle (109) produziert wird, an einer Vorrichtung (101) und einem elektromagnetischen Signal (115), das von einer Fläche (111) eines Körpers (107) reflektiert wird, der die Tonquelle (109) umfasst und der vibriert, wenn der Ton (113) produziert wird; und
Bereitstellen der Zeitdifferenz, um die Bestimmung einer Fluidtemperatur auf Basis der Schallgeschwindigkeit durch das Fluid zwischen dem Körper (107) und der Vorrichtung (101) zu ermöglichen.

2. Einrichtung nach Anspruch 1, wobei die Zeitdifferenz unter Verwendung von einem oder mehreren Merkmalen des Tons (113) und einem oder mehreren Merkmalen eines Signals, das die Vibration der Fläche (111) repräsentiert, die einander entsprechen sollen, bestimmt wird.

3. Einrichtung nach Anspruch 2, wobei das Signal, das die Vibration der Fläche (111) repräsentiert, durch Umwandeln des elektromagnetischen Signals (115), das von der Fläche (111) reflektiert wird, in ein elektrisches Signal und Verarbeiten dieses elektrischen Signals, um vor der Reflexion von der Fläche (111) Frequenzkomponenten des elektromagnetischen Signals (115) herauszufiltern, erhalten wird.

4. Einrichtung nach einem der vorhergehenden Ansprüche, wobei die Zeitdifferenz anhand einer Frequenz eines Signals bestimmt wird, die aus einem Chirp-Mischen unter Verwendung eines Chirps, der aus dem Signal extrahiert wird, das die Vibration der Fläche (111) repräsentiert, und eines entsprechenden Chirps, der aus dem Ton (113) extrahiert wird, resultiert.

5. Einrichtung nach einem der vorhergehenden Ansprüche, wobei die Mittel dazu ausgelegt sind, eine Distanz zwischen dem Körper (107) und der Vorrichtung (101) zu erhalten.

6. Einrichtung nach Anspruch 5, wobei die Distanz durch Verarbeiten des elektromagnetischen Signals (115), das von der Fläche (111) reflektiert wird, erhalten wird.

7. Einrichtung nach einem der vorhergehenden Ansprüche, wobei die Mittel dazu ausgelegt sind zu bewirken, dass einem Benutzer der Vorrichtung (101), die eine relative Zielposition zwischen dem Körper (107) und der Vorrichtung (101) anzeigt, eine Anleitung bereitgestellt wird.

8. Einrichtung nach einem der vorhergehenden Ansprüche, wobei die Mittel dazu ausgelegt sind zu bewirken, dass die Vorrichtung (101) ein elektromagnetisches Signal (115) emittiert, das von der Fläche (111) reflektiert wird, und das elektromagnetische Signal (115), das von der Fläche (111) reflektiert wird, zu detektieren.

9. Vorrichtung nach einem der vorhergehenden Ansprüche, wobei die Mittel dazu ausgelegt sind, den Ton (113) in ein elektrisches Signal umzuwandeln, das den Ton (113) repräsentiert.

10. Einrichtung nach einem der vorhergehenden Ansprüche, wobei der Ton (113) ein Ton ist, der unter Verwendung des Vokaltrakts (409) einer Testperson ist und die Fläche ein Körperteil (411A, B) der Testperson (407) ist, das vibriert, wenn der Ton (113) unter Verwendung des Vokaltrakts (409) der Testperson produziert wird.

11. Einrichtung nach Anspruch 10, wobei die Mittel dazu ausgelegt sind, die Zeitdifferenz bereitzustellen, um auf Basis der Schallgeschwindigkeit durch die Luft zwischen der Testperson (407) und der Vorrichtung (101) die Bestimmung der Ausatmungstemperatur der Testperson zu ermöglichen.

12. Einrichtung nach Anspruch 11, wobei die Mittel dazu ausgelegt sind zu bewirken, dass der Testperson (407) ein Alarm bereitgestellt wird, wenn die Ausatmungstemperatur der Testperson außerhalb eines Schwellwerts liegt, der anhand von Unterlagen von einer oder mehreren zuvor bestimmten Ausatmungstemperaturen bestimmt wird.

13. Einrichtung nach einem der Ansprüche 10 bis 12, wobei die Mittel dazu ausgelegt sind zu bewirken, dass der Testperson (407) eine Anleitung bereitgestellt wird, die einen zu produzierenden Zielton anzeigt.

14. Verfahren, das durch die folgenden Schritte gekennzeichnet ist:
Bestimmen (303) einer Zeitdifferenz zwischen einer Ankunft eines Tons (113), der von einer Tonquelle (109) produziert wird, an einer Vorrichtung (101) und einem elektromagnetischen Signal (115), das von einer Fläche (111) eines Körpers (107) reflektiert wird, der die Tonquelle (109) umfasst und der vibriert, wenn der Ton (113) produziert wird; und
Bereitstellen (305) der Zeitdifferenz, um die Bestimmung einer Fluidtemperatur auf Basis der Schallgeschwindigkeit durch das Fluid zwischen dem Körper (107) und der Vorrichtung (101) zu ermöglichen.

15. Computerprogramm, das Anweisungen umfasst, die, wenn das Programm auf einem Computer ausgeführt wird, bewirken, dass der Computer die folgenden Schritte durchführt:
Bestimmen einer Zeitdifferenz zwischen einer Ankunft eines Tons (113), der von einer Tonquelle (109) produziert wird, an einer Vorrichtung (101) und einem elektromagnetischen Signal (115), das von einer Fläche (111) eines Körpers (107) reflektiert wird, der die Tonquelle (109) umfasst und der vibriert, wenn der Ton (113) produziert wird; und
Bereitstellen der Zeitdifferenz, um die Bestimmung einer Fluidtemperatur auf Basis der Schallgeschwindigkeit durch das Fluid zwischen dem Körper (107) und der Vorrichtung (101) zu ermöglichen.

## Revendications

1. Appareil **caractérisé par** des moyens pour :
déterminer une différence de temps entre l'arrivée, au niveau d'un dispositif (101), d'un son (113) produit par une source sonore (109) et d'un signal électromagnétique (115) réfléchi par une surface (111), d'un corps (107) comprenant la source sonore (109) qui vibre lorsque le son (113) est produit ; et
fournir la différence de temps pour permettre la détermination d'une température de fluide sur la base de la vitesse du son à travers le fluide entre le corps (107) et le dispositif (101).

2. Appareil selon la revendication 1, dans lequel la différence de temps est déterminée en utilisant une ou plusieurs caractéristiques du son (113) et une ou plusieurs caractéristiques d'un signal représentant la vibration de la surface (111) qui sont identifiées pour correspondre les unes aux autres.

3. Appareil selon la revendication 2, dans lequel le signal représentant la vibration de la surface (111) est obtenu en transduisant le signal électromagnétique (115) réfléchi par la surface (111) en un signal électrique et en traitant ce signal électrique pour filtrer des composantes de fréquence du signal électromagnétique (115) avant la réflexion par la surface (111).

4. Appareil selon l'une quelconque des revendications précédentes, dans lequel la différence de temps est déterminée à partir d'une fréquence d'un signal résultant d'un mélange d'une compression d'impulsions utilisant une compression d'impulsions extraite du signal représentant une vibration de la surface (111) et une compression d'impulsions correspondante extraite du son (113).

5. Appareil selon l'une quelconque des revendications précédentes, dans lequel les moyens sont configurés pour obtenir une distance entre le corps (107) et le dispositif (101).

6. Appareil selon la revendication 5, dans lequel la distance est obtenue par traitement du signal électromagnétique (115) réfléchi par la surface (111).

7. Appareil selon l'une quelconque des revendications précédentes, dans lequel les moyens sont configurés pour provoquer la fourniture d'un guidage à un utilisateur du dispositif (101) qui indique une position relative cible entre le corps (107) et le dispositif (101).

8. Appareil selon l'une quelconque des revendications précédentes, dans lequel les moyens sont configurés pour amener le dispositif (101) à émettre un signal électromagnétique (115) qui est réfléchi par la surface (111) et à détecter le signal électromagnétique (115) réfléchi par la surface (111).

9. Appareil selon l'une quelconque des revendications précédentes dans lequel les moyens sont configurés pour transduire le son (113) en un signal électrique représentant le son (113).

10. Appareil selon l'une quelconque des revendications précédentes, dans lequel le son (113) est un son produit en utilisant un conduit vocal (409) d'un sujet et la surface est une partie de corps (411A, B) du sujet (407) qui vibre lorsque le son (113) est produit en utilisant le conduit vocal (409) du sujet.

11. Appareil selon la revendication 10, dans lequel les moyens sont configurés pour fournir la différence de temps pour permettre la détermination de la température d'haleine expirée du sujet sur la base de la vitesse du son dans l'air entre le sujet (407) et le dispositif (101).

12. Appareil selon la revendication 11, dans lequel les moyens sont configurés pour provoquer la fourniture d'une alerte au sujet (407) si la température d'haleine expirée du sujet est en dehors d'un seuil déterminé à partir d'un enregistrement d'une ou plusieurs températures d'haleine expirée déterminées précédemment.

13. Appareil selon l'une quelconque des revendications 10 à 12, dans lequel les moyens sont configurés pour provoquer la fourniture d'un guidage au sujet (407) qui indique un son cible à produire.

14. Procédé **caractérisé par** les étapes suivantes :
déterminer (303) une différence de temps entre l'arrivée, au niveau d'un dispositif (101), d'un son (113) produit par une source sonore (109) et d'un signal électromagnétique (115) réfléchi par une surface (111), d'un corps (107) comprenant la source sonore (109) qui vibre lorsque le son (113) est produit ; et
fournir (305) la différence de temps pour permettre la détermination d'une température de fluide sur la base de la vitesse du son à travers le fluide entre le corps (107) et le dispositif (101).

15. Programme informatique comprenant des instructions qui, lorsque le programme est exécuté sur un ordinateur, amènent l'ordinateur à réaliser les étapes suivantes :
déterminer une différence de temps entre l'arrivée, au niveau d'un dispositif (101), d'un son (113) produit par une source sonore (109) et d'un signal électromagnétique (115) réfléchi par une surface (111), d'un corps (107) comprenant la source sonore (109) qui vibre lorsque le son (113) est produit ; et
fournir la différence de temps pour permettre la détermination d'une température de fluide sur la base de la vitesse du son à travers le fluide entre le corps (107) et le dispositif (101).
